# FASCICULE DE BREVET EUROPEEN

(11) **EP 2 702 949 B1**
(45) Date de publication et mention de la délivrance du brevet: **29.07.2015**
(21) Numéro de dépôt: 13182217.3
(22) Date de dépôt: 29.08.2013
(51) Int. Cl.: A61B 10/00, B01J 20/28, G01N 1/40

(54) **Dispositif et système de prélèvement pour capture de cibles biologiques d'un fluide corporel, et procédé de fabrication de ce dispositif**
Probenentnahmevorrichtung und -system zum Erfassen einer biologischen Probe in einer Körperflüssigkeit, und die Methode zur Herstellung
Sampling device and system for capturing biological samples in a bodily fluid, and method of manufacturing same

(30) Priorité: 30.08.2012 FR 1258114
(43) Date de publication de la demande: 05.03.2014
(73) Titulaire: COMMISSARIAT A L'ENERGIE ATOMIQUE ET AUX ENERGIES ALTERNATIVES, 75015 Paris (FR)
(72) Inventeur: Cosnier, Marie-Line, 38000 GRENOBLE (FR); Lauro, Dominique, 38580 LE MOUTARET (FR)
(74) Mandataire: Bolinches, Michel Jean-Marie

(56) Documents cités:
- WO-A2-2005/123952
- FR-A1- 2 955 024

## Description

La présente invention concerne un dispositif de prélèvement adapté pour être inséré dans un embout tubulaire creux de type aiguille ou cathéter et pour déboucher hors de l'embout en vue d'une mise en contact avec un fluide corporel contenant des cibles biologiques à prélever, un système de prélèvement incorporant cet embout et ce dispositif qui y est monté coulissant, et un procédé de fabrication de ce dispositif. L'invention s'applique à des prélèvements réalisés en particulier *in vivo* dans des fluides corporels du corps humain, par exemple des fluides corporels circulants, et notamment le sang, le liquide céphalorachidien, le fluide interstitiel ou la lymphe, ces fluides pouvant contenir à titre de cibles des protéines, des oligonucléotides tels que de l'ARN ou de l'ADN, des anticorps, des enzymes ou des cellules, à titre non limitatif.

Depuis de nombreuses années, les techniques d'analyse, qu'elles soient basées sur la génomique, la protéomique ou l'immunologie, progressent et atteignent des niveaux de sensibilité remarquables. Ces techniques sont basées sur la reconnaissance d'éléments d'intérêt ou cibles biologiques qu'il faut extraire des autres éléments présents dans le prélèvement effectué que cela soit fait *in vivo* ou *ex vivo.* Si la cible est absente ou en trop faible proportion par rapport à la sensibilité de la méthode d'analyse, alors la mesure ne sera pas possible.

Les méthodes de préparation de l'échantillon visent à capturer les cibles recherchées et à les mettre en contact après concentration avec une surface fonctionnalisée, sur laquelle on réalise une mesure. Elles sont très intéressantes car elles permettent, par concentration de cette cible, de relâcher la contrainte sur la sensibilité de mesure. Par contre, elles sont inopérantes si la cible n'est pas présente dans le prélèvement effectué. Cet écueil est évident par exemple, lors de l'analyse sanguine qui a vu la course à la réduction du volume prélevé mise à mal par la présence ou non de l'élément recherché et par la sensibilité du système de mesure.

Une méthode classique est de mélanger des nanoparticules porteuses de sites de reconnaissance, telles que des nanobilles, avec l'échantillon contenant la cible puis d'effectuer la reconnaissance en volume et enfin de récupérer ces nanoparticules par centrifugation ou attraction magnétique avant d'effectuer un re-largage contrôlé des cibles captées sur une surface de mesure.

Une autre méthode connue consiste à effectuer une re-circulation du liquide à tester sur une surface présentant les sites de reconnaissance en question.

La problématique du test de fluides corporels circulant dans le corps humain peut s'appréhender de la même manière. Dans l'exemple où le volume à tester est l'ensemble du sang ou du liquide céphalorachidien, on peut par analogie envisager la même approche qui consiste à injecter des nanoparticules métalliques et/ou magnétiques dans le fluide corporel ou sous la peau, les laisser reconnaître les cibles puis les récupérer par exemple par apposition d'un champ magnétique local ou par filtration sur une dérivation extra-corporelle. Cette approche nécessite une étude très approfondie des particules injectées en termes de toxicité et de filtration dans les reins, le foie, etc. Un problème non résolu à ce jour est une récupération satisfaisante de telles particules injectées.

Pour parer aux problèmes notamment de risque de déclenchement de réactions immunitaires et de toxicité que peut poser l'injection de telles nanoparticules dans le corps humain, les solutions développées à ce jour reposent en général sur l'encapsulation de ces nanoparticules métalliques et/ou magnétiques dans différents matériaux et en particulier dans des polymères biocompatibles. En fonction de la porosité de ce polymère, il peut faire office de filtre, bloquant des espèces biologiques dépassant une certaine taille. Par espèce biologique, on entend ici les cellules, les molécules, virus, bactéries ou anticorps.

Dans les dispositifs connus, l'utilisation d'un revêtement ou d'une encapsulation améliore les performances du dispositif de mesure que ce soit pour l'efficacité de contraste, la tolérance face aux tissus ou la capacité de capture. Cependant, un inconvénient majeur de ces dispositifs est qu'ils ne sont pas conçus pour récupérer les nanoparticules après leur mise en contact avec les cibles recherchées dans le fluide considéré, notamment pour la capture de ces cibles ou à la suite d'une injection préalablement à une analyse par IRM (imagerie par résonance magnétique). En d'autres termes, se pose le problème dans ces dispositifs connus du devenir des nanoparticules injectées *in vivo*.

Le document FR 2 955 024 au nom de la Demanderesse permet de remédier à cet inconvénient, en présentant un dispositif de mise en contact transitoire de supports de capture de cibles à analyser avec un fluide corporel les contenant, ce dispositif comprenant un embout de prélèvement dont une extrémité de mise en contact avec le fluide est liée aux supports de capture qui sont recouverts par une couche polymérique réticulée biocompatible et poreuse. Cette couche est conçue pour retenir les supports de capture et pour ne laisser passer que des particules incluant ces cibles de taille inférieure à une taille limite, de sorte que les supports de capture soient récupérables après la mise en contact par dissolution de cette couche. Ce document mentionne l'intégration possible des supports de capture à une structure maillée reliée à l'embout dont les brins sont recouverts de ladite couche, laquelle encapsule ces supports de capture qui peuvent être constitués de nanoparticules fonctionnalisées. Cette structure maillée est repliée dans une structure de guidage par exemple tubulaire et déployée réversiblement hors de celle-ci lors du prélèvement. La structure maillée est destinée à être déployée dans l'organisme, par exemple dans un vaisseau sanguin, de la même façon qu'un « stent ». Il est donc clair que le fluide biologique s'écoule entre les brins, c'est-à-dire entre les mailles du dispositif.

Bien que les dispositifs présentés dans ce document procurent des résultats tout à fait satisfaisants, la Demanderesse a cherché à optimiser l'accrochage ou adhésion de l'ensemble de la couche polymérique à une telle structure maillée, de sorte à minimiser les risques de détachement de fragments polymériques vis-à-vis de la surface que cette couche recouvre.

Le document WO-A1-2010/145824 présente un dispositif de détection pour l'enrichissement d'échantillons, comprenant une surface de détection tridimensionnelle qui est rendue fonctionnelle par une multitude de récepteurs de détection qu'elle présente et qui peut être microstructurée par exemple à la manière d'un réseau maillé. Ce document ne concerne pas l'adhérence à une armature d'une couche polymérique enrobant des supports de capture distincts de cette armature.

Un but de la présente invention est de proposer un dispositif de prélèvement adapté pour être inséré dans un embout tubulaire creux de type aiguille ou cathéter et pour déboucher hors de l'embout en vue d'une mise en contact notamment *in vivo* avec un fluide corporel contenant des cibles biologiques à prélever, qui remédie aux inconvénients précités, le dispositif comprenant une armature microstructurée par des ajours, et une couche polymérique réticulée biocompatible et poreuse, qui comporte des supports de capture aptes à capturer lesdites cibles et qui est adaptée pour retenir ces supports vis-à-vis du fluide et pour ne laisser passer que des particules du fluide incluant ces cibles de taille inférieure à une taille limite.

A cet effet, un dispositif selon l'invention est tel que ladite couche polymérique remplit tout ou partie desdits ajours, de sorte à être retenue par ladite armature.

Selon un mode de réalisation de l'invention, l'armature est inscrite dans au moins une surface cylindrique de plus grande dimension transversale comprise entre 500 µm et 2 mm, l'armature étant noyée dans ladite couche polymérique.

Autrement dit, la couche polymérique remplit tout ou partie des ajours de l'armature, en s'étendant de part et d'autre de cette dernière.

L'armature peut comprendre une face externe et délimiter un volume interne, la couche polymérique s'étendant à travers lesdits ajours de ce volume interne à cette face externe et au-delà de cette dernière.

L'armature peut être tubulaire, auquel cas elle enveloppe un volume interne cylindrique, ladite couche polymérique s'étendant alors de part et d'autre de l'armature. On notera que cette armature globalement tubulaire microstructurée par des ajours (i.e. par des micro-ouvertures traversantes de dimensions de l'ordre d'une ou de plusieurs dizaines de µm) laissent passer le matériau polymérique non réticulé (i.e. non encore gélifié) de la couche poreuse de sorte à remplir en partie ou en totalité l'espace interne de cette armature, ce qui permet d'améliorer de manière significative l'accrochage de cette couche à l'armature par cette adhérence optimisée sur les deux faces radialement interne et externe de l'armature. Il en résulte que les risques de décrochage de la couche vis-à-vis de l'armature et donc de libération d'un fragment de cette couche dans le fluide corporel siège du prélèvement sont minimisés.

Par « embout », on entend dans la présente description un embout pouvant correspondre à tout ou partie d'une aiguille d'injection ou de prélèvement, d'un cathéter ou d'un système externe, qui est apte à être introduit dans le milieu contenant le fluide corporel. Un tel milieu peut par exemple être la veine d'un être humain ou animal.

Par « au moins une surface cylindrique », on entend dans la présente description une ou plusieurs surfaces cylindriques (i.e. chacune étant définie sur sa hauteur par une génératrice et sur sa section transversale par une directrice en forme de ligne courbe quelconque, par exemple une directrice en forme d'ellipse ou de cercle), étant précisé que la section transversale de la face externe de l'armature peut être constante (cas où cette face est inscrite dans une seule surface cylindrique) ou bien variable (cas où cette face est successivement inscrite dans plusieurs surfaces cylindriques et/ou dans une ou plusieurs surfaces tronconiques succédant à une ou plusieurs surfaces cylindriques).

La couche réticulée du dispositif selon l'invention présente une perméabilité sélective et permet d'éviter un contact direct entre les supports de capture et le fluide afin de prévenir une réaction immunitaire, d'empêcher la diffusion de tout ou partie des supports de capture dans le fluide, de capturer sélectivement les cibles selon leur taille et d'imposer moins de contraintes mécaniques aux tissus biologiques environnants du fait de sa souplesse.

Selon une autre caractéristique de l'invention, ladite armature peut présenter un unique axe longitudinal de symétrie qui est destiné à être parallèle à celui dudit embout, ladite armature conservant une géométrie globalement tubulaire dans ses positions où elle est insérée dans l'embout et où elle débouche hors de ce dernier.

En d'autres termes et contrairement à la structure maillée du document WO-A1-2011/086486 précité que l'on déploie hors de l'embout et que l'on replie dans ce dernier, une armature selon l'invention est inapte à être déployée hors de l'embout (i.e. étendue ou ouverte, par exemple déroulée) et repliée dans ce dernier (i.e. escamotée de manière plus compacte, par exemple enroulée) du fait qu'elle est sensiblement indéformable et présente une géométrie de révolution épousant la face interne de la zone d'extrémité correspondante de l'embout.

Selon une autre caractéristique de l'invention, la couche polymérique réticulée peut présenter une viscosité, mesurée par un rhéomètre cône - plan, qui est égale ou supérieure à 100 mPa.s et de préférence comprise entre 150 mPa.s et 5800 mPa.s. Au-delà de ces valeurs, la viscosité est trop élevée, ce qui limite les possibilités de mise en forme du polymère.

On notera que cette viscosité particulière de la couche polymérique contribue de manière significative au bon accrochage de cette couche sur l'armature microstructurée du dispositif selon l'invention qui est caractérisée par les irrégularités et aspérités de surface formées par les bords radialement externes et internes des micro-ajours.

Avantageusement, cette armature micro-ajourée d'un dispositif selon l'invention peut être de type toile grillagée, tissée ou tressée, comprenant une multitude d'ouvertures ou interstices séparés deux à deux suivant un pas compris entre 30 µm et 60 µm.

Selon un exemple particulièrement avantageux de réalisation de l'invention, ladite armature est de type toile métallique, tissée ou grillagée. Elle peut alors être sensiblement plane, ou former une surface fermée de façon à former un volume interne. Elle peut alors comprendre un ou plusieurs tubes cylindriques micro-grillagés et de section sensiblement circulaire avec, dans le cas de plusieurs tubes, leurs axes longitudinaux respectifs de symétrie qui sont parallèles. Il peut s'agir de tubes concentriques.

De préférence, ladite armature présente une épaisseur comprise entre 10 µm et 100 µm, et ladite au moins une surface cylindrique présente une section sensiblement elliptique ou circulaire.

On notera toutefois qu'une armature selon l'invention peut présenter en outre, sur ses faces externe et/ou interne, des zones non ajourées mais microstructurées d'une autre manière, par exemple par des creux (i.e. des trous non radialement traversants) et/ou des reliefs continus ou discontinus de dimensions - telles que la profondeur radiale - inférieures à 1 mm, ces creux et/ou reliefs présentant de préférence de telles dimensions comprises entre 20 µm et 90 µm sur ces faces externe et/ou interne.

En particulier, une telle armature microstructurée par ces creux et/ou reliefs en plus desdits micro-ajours peut être obtenue par un sablage d'une ou plusieurs zones de sa face externe initialement lisse(s).

Selon une autre caractéristique de l'invention, ladite couche polymérique peut former par rapport à ladite face externe de l'armature un revêtement externe sensiblement coaxial à cette armature et d'épaisseur comprise entre 50 µm et 300 µm.

Selon l'invention, ladite armature peut être noyée dans ladite couche polymérique sur une partie de sa longueur axiale comprise entre 1 mm et 5 cm, cette couche polymérique pouvant avantageusement présenter un volume compris entre 1 mL et 10 mL.

On notera qu'un tel volume permet de renfermer une grande quantité de supports de capture, tels que des nanobilles, ce qui est particulièrement utile lorsqu'on souhaite capturer des espèces minoritaires circulant dans un liquide corporel.

Selon une autre caractéristique de l'invention, lesdits supports de capture peuvent comprendre des nanoparticules magnétiques ou non qui sont fonctionnalisées en surface par des fonctions greffées aptes à capturer lesdites cibles, qui présentent une plus grande dimension transversale comprise entre 50 nm et 500 nm et qui sont noyées dans la masse de ladite couche polymérique, ces nanoparticules étant de préférence des nanobilles ou nanosphères à base d'un oxyde de fer de diamètre compris entre 80 nm et 200 nm et fonctionnalisées par des fonctions anioniques ou cationiques (ou en variante par des anticorps, des oligonucléotides tels que les aptamères, des fonctions de surfaces de type chromatographie et les fonctions des librairies peptidiques et oligonucléotidiques).

Avantageusement, l'armature peut être réalisée en un matériau métallique, de préférence de l'acier inoxydable de grade chirurgical, en silicium ou en une matière polymérique telle qu'un silicone, et la couche polymérique est à base d'au moins un polymère biocompatible et à gélification réversible choisi dans le groupe constitué par les gels d'alginate, les copolymères d'alginate et de poly-L-lysine, le chitosan, l'agarose, la cellulose, le poly(triméthylammonium éthylacrylate méthyl sulfate)-b-poly (acrylamide), le poly(hydroxyéthylmétacrylate (HEMA), le poly(hydroxyéthylmétacrylate-méthyl éthacrylate (HEMA-MMA) et les autres copolymères à base de méthacrylate, les polyéthylèneglycols, les copolymères d'acrylonitrile et de polyéthylèneglycol, les polysaccharides et leurs mélanges.

Encore plus avantageusement, l'armature peut être pourvue à sa surface de groupements fonctionnels créant des liaisons chimiques entre l'armature et la couche polymérique, de préférence des groupements acide carboxylique ou amine dans le cas où l'armature est métallique pour une liaison avec des groupements hydroxyle de cette couche.

De préférence, la couche polymérique est à base d'au moins un gel d'alginate qui est obtenu au moyen de polycations de préférence choisis dans le groupe constitué par les polycations de calcium, baryum, fer et strontium. L'utilisation d'un alginate est en effet particulièrement avantageuse, du fait qu'il est parfaitement biocompatible, non toxique et laisse les cibles à capturer le traverser. De plus, il est polymérisable et gélifiable aux températures ambiantes et demeure sous forme gélifiée à des températures corporelles et au pH correspondant aux conditions physiologiques.

Egalement avantageusement, la couche polymérique réticulée peut présenter un module d'Young, mesuré à partir de tests de compression réalisés avec un rhéomètre, inclusivement compris entre 50 kPa et 270 kPa. Au-delà de ces valeurs, le polymère devient difficile à mettre en forme.

Avantageusement, la couche polymérique peut présenter une porosité définissant ladite taille limite qui est comprise entre 10 nm et 1 µm, avec une porosité en surface comprise entre 10 nm et 50 nm et une porosité dans la masse comprise entre 100 nm et 1 µm dans l'exemple préférentiel d'un gel d'alginate.

Un système de prélèvement selon l'invention comprend :
- un embout tubulaire creux de type aiguille ou cathéter qui présente un diamètre interne compris entre 500 µm et 2 mm,
- un dispositif de prélèvement inséré dans l'embout et apte à déboucher par coulissement hors d'une extrémité de cet embout en vue d'une mise en contact notamment *in vivo* avec un fluide corporel contenant des cibles biologiques à prélever, et
- un organe de poussée apte à faire coulisser réversiblement ledit dispositif de prélèvement hors dudit embout.

Ce système de l'invention est **caractérisé en ce que** ce dispositif est tel que défini ci-dessus et est optionnellement pourvu d'un moyen de liaison à ladite extrémité de l'embout.

Selon une autre caractéristique de l'invention, ledit organe de poussée peut être de type seringue, et comporte :
- un corps de pompe dans lequel est monté ledit embout, et
- une tige insérable dans ledit embout pour y faire coulisser ledit dispositif de prélèvement suivant une translation réversible parallèle à l'axe de symétrie de l'embout.

De préférence, on dispose un liquide, de type liquide physiologique, entre ladite tige insérable et ledit dispositif de prélèvement. Cela permet de limiter la dégradation du polymère lors de la poussée. Le liquide est avantageusement injectable.

Un procédé de fabrication selon l'invention d'un dispositif de prélèvement tel que celui défini ci-dessus comprend les étapes suivantes :
a) préparation d'un composite polymérique non réticulé incorporant lesdits supports de capture et ladite couche polymérique les recouvrant à l'état non réticulé,
b) insertion de ladite armature dépourvue de ce composite dans un moule tubulaire, avec optionnellement une liaison de l'armature à une extrémité de prélèvement de l'embout (de préférence, le moule est perforé de telle sorte que la solution de gélification est en contact avec la périphérie du polymère durant la gélification de ce dernier, cela permettant de mieux maîtriser la porosité de surface ; la taille des perforations est avantageusement inférieure à 1 mm).
c) assemblage de l'embout contenant cette armature dans un organe de prélèvement de type seringue,
d) prélèvement du composite non réticulé préparé en a) par cet organe de prélèvement, pour injecter ce composite à l'intérieur de l'embout au contact de l'armature, puis
e) réticulation dans un bain gélifiant de l'embout qui est rempli du composite non réticulé injecté en d) et que l'on a préalablement extrait de cet organe de prélèvement, pour l'obtention de ladite couche polymérique réticulée solidaire de l'armature.

Avantageusement, l'étape a) peut comprendre :
a1) une dispersion dans une solution tampon aqueuse desdits supports de capture comprenant des nanoparticules fonctionnalisées magnétiques ou non, puis
a2) un ajout sous agitation à la dispersion obtenue en a1) d'au moins un polymère biocompatible et à gélification réversible, pour l'obtention du composite non réticulé dans lequel sont noyées ces nanoparticules.

D'autres avantages, caractéristiques et détails de l'invention ressortiront du complément de description qui va suivre en référence à des dessins annexés, donnés uniquement à titre d'exemples et parmi lesquels :
la figure 1 est une vue schématique illustrant différentes phases d'une méthode de prélèvement de cibles biologiques dans un fluide corporel selon un exemple de réalisation de l'invention,
la figure 2 est une vue partielle de face d'une installation pour la préparation d'un composite polymérique réticulé inclus dans un dispositif de prélèvement selon l'invention,
la figure 3 est une vue schématique en coupe longitudinale d'un embout de type aiguille qui contient un dispositif de prélèvement selon l'invention et que l'on a introduit dans une veine du corps humain, le dispositif étant en position rentrée dans l'embout,
la figure 4 est une vue schématique en coupe longitudinale de l'embout de la figure 3 toujours introduit dans cette veine, mais le dispositif de prélèvement étant en position partiellement sortie par rapport à l'embout,
la figure 5 est une vue de détail d'une armature micro-ajourée d'un dispositif de prélèvement selon un exemple de l'invention, cette armature étant dépourvue du composite polymérique réticulé destiné à la recouvrir,
la figure 6 est une photographie montrant le recouvrement de l'armature de la figure 5 par ce composite réticulé,
la figure 7 est une vue partielle en plan d'un embout de type aiguille contenant une armature filaire non conforme à l'invention dépourvue de ce composite réticulé,
la figure 8 est une vue partielle en plan d'un embout de type aiguille contenant une armature micro-ajourée selon un autre exemple de l'invention dépourvue de ce composite réticulé,
la figure 9 est une photographie montrant le recouvrement par ce composite réticulé de l'armature filaire de la figure 7, immédiatement après injection et réticulation du composite dans l'embout au contact de l'armature,
la figure 10 est une photographie montrant le recouvrement par ce composite réticulé de l'armature micro-ajourée de la figure 8, immédiatement après injection et réticulation du composite dans l'embout au contact de l'armature,
la figure 11 est une photographie montrant le recouvrement par ce composite réticulé de l'armature filaire de la figure 7, après lavage à l'eau du dispositif de prélèvement visible à la figure 9, et
la figure 12 est une photographie montrant le recouvrement par ce composite réticulé de l'armature micro-ajourée de la figure 8, après lavage à l'eau du dispositif de prélèvement visible à la figure 10.

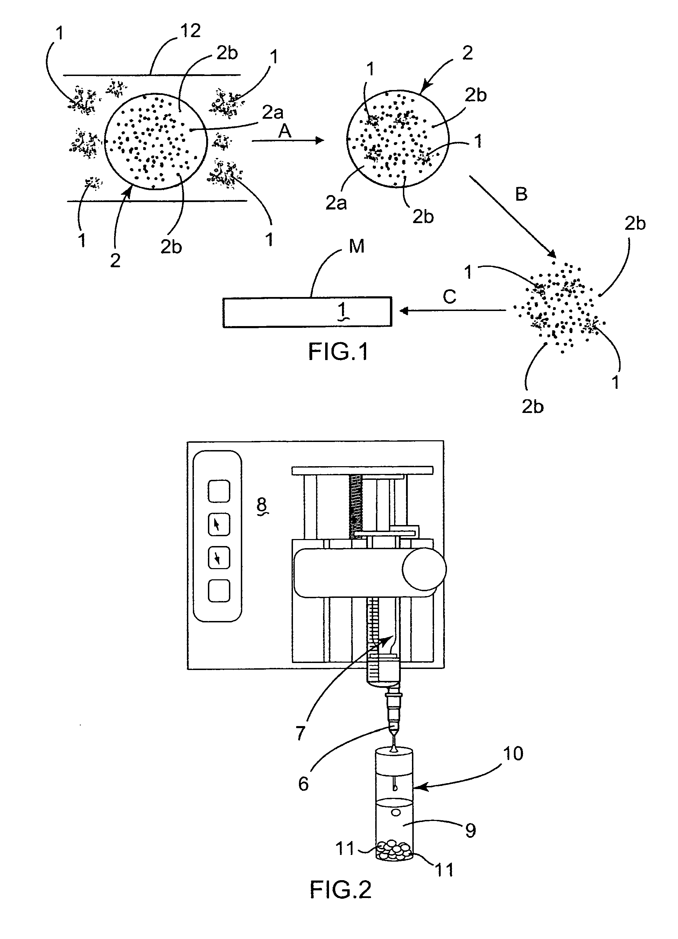

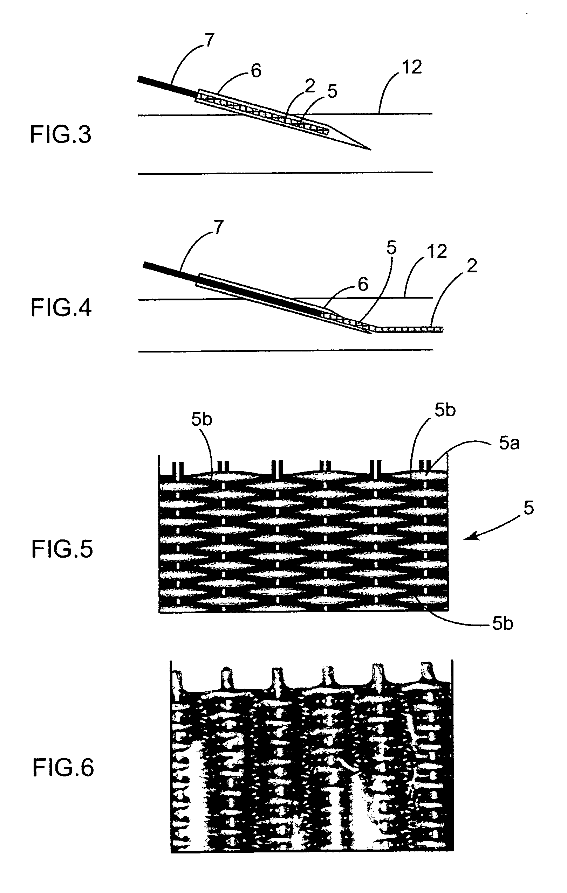

Dans la méthode de prélèvement illustrée à la figure 1, on procède dans une première étape A à une incubation, dans un fluide contenant des cibles 1 (e.g. des protéines), d'un composite polymérique 2 réticulé (i.e. gélifié) constitué dans cet exemple d'une couche 2a d'un gel d'alginate de calcium dans lequel sont noyés des supports de capture 2b formés de nanoparticules magnétiques avantageusement à base de Fe₂O₃ (ce composite 2 est solidaire d'une armature micro-ajourée selon l'invention, non représentée ici et décrite ci-dessous en référence aux figures 5, 6, 8, 10 et 12). Après cette incubation, il se produit par l'effet de filtre du gel d'alginate une pénétration des cibles 1 dans la couche 2a jusqu'à ce que celles-ci soient capturées par les nanoparticules 2b. Ces dernières présentent un diamètre moyen d'environ 100 nm et peuvent être fonctionnalisées ou greffées par des fonctions de surface, par exemple de type polystyrène sulfonate.

On a ensuite procédé à des opérations B de lavage et de dé-gélification de la couche d'alginate 2a, qui ont conduit à l'obtention des nanoparticules 2b liées aux cibles 1 dans l'alginate en solution, avantageusement au moyen d'un agent chélateur de polycations qui est par exemple, pour des polycations sodium, l'acide éthylène diamine tétra acétique (EDTA) ou le citrate de sodium.

On a enfin procédé à une séparation C, avantageusement par aimantation (l'aimant utilisé M est symbolisé par un rectangle à la figure 1), des nanoparticules 2b liées aux cibles 1 qui permet d'obtenir, en vue d'analyses ultérieures, ces cibles 1 telles que des protéines adsorbées sur la surface de capture des nanoparticules 2b.

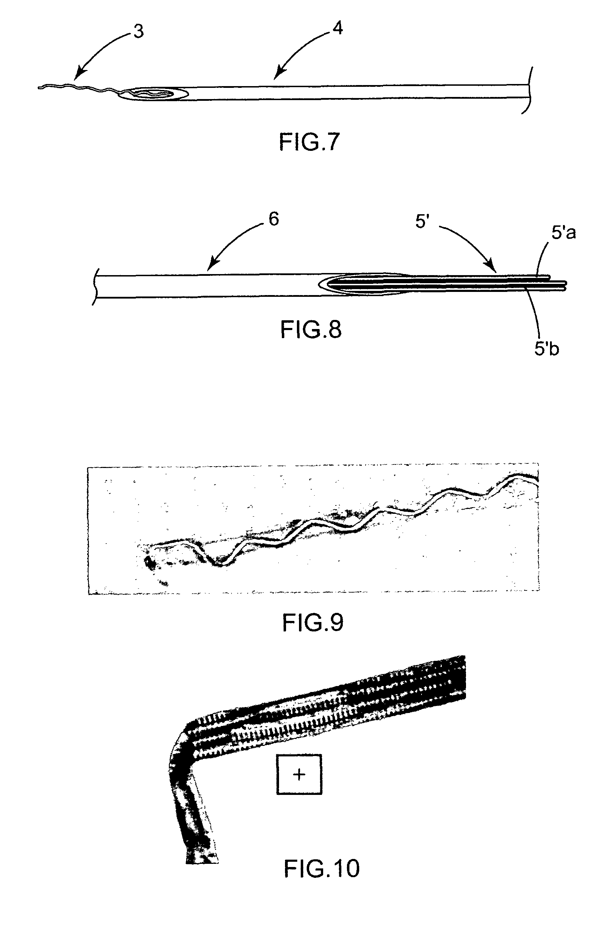

On a préparé de la manière suivante des composites polymériques 2 incorporant les nanoparticules 2b noyées dans la couche polymérique poreuse 2a. On a commencé par ajouter et disperser par agitation ces nanoparticules 2b dans un tampon aqueux composé de 154 mM de NaCl et d'HEPES. Puis on a ajouté à la dispersion ainsi obtenue un alginate en poudre selon une fraction massique variant de 1 % à 3 %, et en particulier égale à 1,5 % pour les deux exemples de l'invention relatifs aux figures 6 et 10), avec agitation rotative et sous ultrasons pendant au moins 10 heures. On a ainsi obtenu les composites 2 à l'état non réticulé constitués d'un hydrogel d'alginate polymérisé 2a enrobant les nanoparticules 2b.

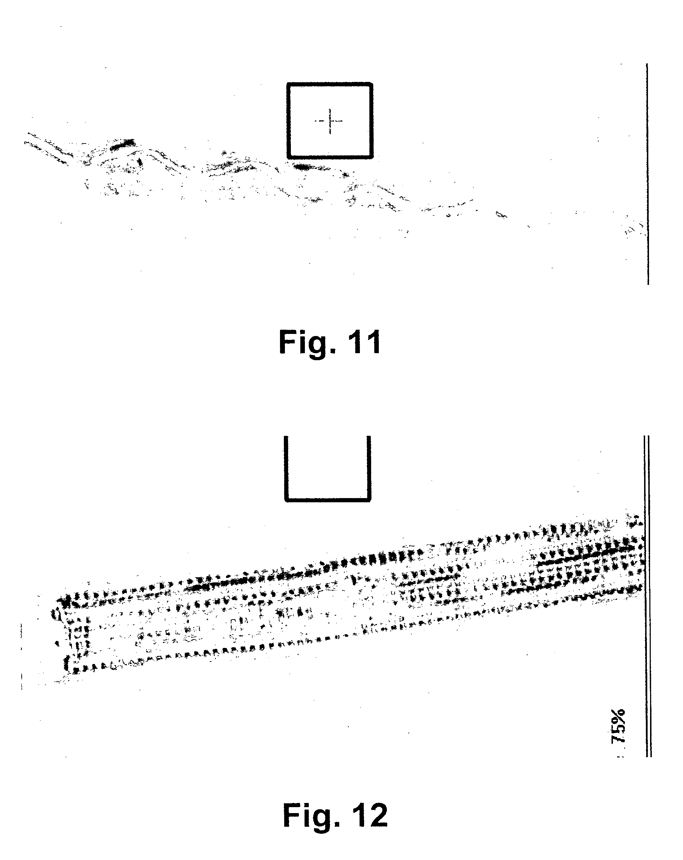

Puis, indépendamment des composites 2 non gélifiés ainsi préparés, on a inséré :
- dans l'exemple non conforme à l'invention de la figure 7, une armature 3 constituée d'un unique fil métallique de diamètre 140 µm (en acier inoxydable chirurgical AISI 316L) dans une aiguille 4 de diamètre extérieur égal à 0,8 mm,
- dans l'exemple selon l'invention de la figure 5, une armature 5 (dont seule la face radialement externe 5a est visible) formée d'une toile tissée ou tressée (en acier inoxydable chirurgical AISI 316L) globalement tubulaire présentant suivant un pas d'environ 50 µm des interstices 5b à travers lesquels va pénétrer le composite 2 pour revêtir en outre la face radialement interne de l'armature 5, dans une aiguille 6 de diamètre extérieur égal à 1,1 mm, et
- dans l'autre exemple selon l'invention des figures 8, 10, 12, une armature 5' microgrillagée (également en acier inoxydable chirurgical AISI 316L) dans une même aiguille 6, (les photographies des figures 10 et 12 permettent de mieux distinguer la structure micro-grillagée de cette armature 5', laquelle s'étend sur une surface 5'a et présente des ajours 5'b comme visible à la figure 8).

Dans ces trois cas de figure, les armatures 3, 5, 5' sont inscrites dans un volume cylindrique de plus grande dimension transversale comprise entre 500 µm et 5 mm.

On a assemblé chacune de ces aiguilles 4, 6 dans une seringue 7 (voir figure 2) de 5 mL munie d'un dispositif pousse-seringue 8 et l'on a injecté via cette seringue 7 le composite 2 à gélifier dans l'aiguille 4, 6 de sorte que ce composite 2 recouvre l'armature correspondante 3, 5, 5'. Une fois l'aiguille 4, 6 remplie du composite 2, on a retiré la seringue 7 et l'on a plongé cette aiguille 4, 6 dans un bain gélifiant aqueux 9 à base de polycations de préférence calcium (16 mM NaCl, 20 mM CaCl₂). Comme visible à la figure 2, on a par exemple réalisé la gélification du composite 2 par ajout goutte-à-goutte avec ce bain gélifiant 9 contenu dans un flacon 10, et l'on a ainsi obtenu au bout d'un temps relativement long (de préférence pendant au moins trois jours) des billes 11 de gel d'alginate réticulé de diamètre moyen compris entre 2 mm et 3 mm.

On a ainsi obtenu des « boudins » de composite 2 recouvrant l'armature 3, 5, 5' sur une partie de la longueur axiale de celle-ci, cette longueur de recouvrement étant comprise entre 1 cm et 5 cm. Les photographies des figures 9 et 10 montrent ces recouvrements, étant précisé que pour une meilleure visualisation du composite 2, ces photographies ont été prises avec la couche d'alginate 2a seule sans les nanoparticules 2b qui auraient assombri le composite 2 en empêchant de distinguer l'armature 3, 5'.

Ainsi, les armatures 5, 5' présentant des ajours 5b, 5'b sont partiellement noyées dans le composite 2, ce dernier s'étendant de part et d'autre de ces armatures 5, 5' à travers ces ajours 5b, 5'b. L'armature 5 délimite un volume interne tubulaire rempli de composite 2, lequel s'étend au-delà dudit volume interne à travers les ajours 5b.

Pour réaliser un prélèvement de cibles 1 dans le fluide corporel 12, on assemble à nouveau la seringue 7 sur l'aiguille 6 remplie du composite 2 réticulé recouvrant l'armature 3, 5, 5' afin de réaliser l'insertion de l'aiguille 6 dans la veine 12, comme illustré à la figure 3 dans laquelle l'armature 5 et le composite 2 la recouvrant ont été représentés schématiquement en position rentrée (i.e. en deçà de l'extrémité ouverte de l'aiguille 6). Après que l'aiguille 6 a pénétré dans la veine 12, on pousse en translation l'armature 5 recouverte du composite 2 le long de l'aiguille 6, de telle sorte qu'elle débouche au-delà de l'extrémité de cette dernière dans la position partiellement sortie de la figure 4 et qu'ainsi les nanoparticules 2b noyées dans le composite 2 soient en contact avec les cibles 1 contenues dans la veine 12. Un liquide, de préférence injectable, peut être disposé entre le moyen de poussée et l'armature 5, 5', afin de préserver le composite 2 lors de la poussée.

Comme illustré à la photographie de la figure 6, on notera que le composite 2 (visible en brillance) recouvre les armatures 5, 5' selon l'invention, d'une part, sur leurs faces 5a, 5'a, mais également sur leurs faces situées de l'autre côté de l'armature. Ces armatures 5, 5' présentent une épaisseur comprise entre 10 µm et 100 µm.

On a réalisé des essais comparatifs de lavage à l'eau des composites 2 recouvrant respectivement l'armature filaire 3 des figures 7 et 9, l'armature 5 selon les figures 5 et 6 et l'armature 5' selon les figures 8 et 10. On a utilisé pour le lavage de ces trois composites 2 une douchette projetant de l'eau déionisée (eau « DI ») pendant une même durée d'une minute.

Les photographies des figures 11 et 12 rendent compte des résultats obtenus pour ces armatures 3 et 5'. Comme visible notamment dans la portion inférieure droite de la figure 11, le gel d'alginate s'est partiellement détaché de l'armature 3 non conforme à l'invention, alors que la figure 12 montre au contraire que le gel d'alginate du composite 2 a bien résisté à ce lavage et recouvre toujours la face externe de l'armature 5' suivant une épaisseur homogène sur toute la longueur axiale de cette armature 5'. Il en est de même pour l'armature 5 des figures 5 et 6.

Ces essais comparatifs montrent donc que les risques de libération de fragments de polymère du composite 2 dans le liquide corporel sont réellement minimisés, grâce aux armatures micro-ajourées 5, 5' selon l'invention.

On notera que le composite 2 réticulé, qui recouvre l'armature 5, 5' selon les exemples de réalisation des figures 5, 6, 8, 10, 12 sur une partie de sa longueur axiale, a été obtenu en utilisant la fraction massique préférentielle précitée de 1,5 % d'alginate dans la dispersion aqueuse. La viscosité de ce composite 2 réticulé constitué de la couche d'alginate 2a à 1,5 % dans laquelle sont noyées les nanoparticules 2b était d'environ 250 mPa.s (viscosité mesurée par un rhéomètre cône-plan), et son module d'Young (calculé à partir de tests de compression réalisés avec un rhéomètre) était d'environ 77 kPa, ce qui permet de contribuer au bon accrochage du composite 2 sur l'armature microstructurée 5, 5'.

## Revendications

1. Dispositif de prélèvement (5 ou 5', 2) adapté pour être inséré dans un embout tubulaire creux (6) de type aiguille ou cathéter, et pour déboucher hors de l'embout en vue d'une mise en contact notamment *in vivo* avec un fluide corporel (12) contenant des cibles biologiques (1) à prélever, le dispositif comprenant :
- une armature (5, 5') microstructurée par des ajours (5b, 5'b), et
- une couche polymérique réticulée (2a) biocompatible et poreuse, qui comporte des supports de capture (2b) aptes à capturer lesdites cibles et qui est adaptée pour retenir ces supports vis-à-vis du fluide et pour ne laisser passer que des particules du fluide incluant ces cibles de taille inférieure à une taille limite,
ladite couche polymérique remplissant tout ou partie desdits ajours, de sorte à être retenue par ladite armature,
**caractérisé en ce que** ladite armature est sensiblement indéformable entre des positions où elle est insérée dans l'embout et où elle débouche hors de ce dernier.

2. Dispositif de prélèvement (5 ou 5', 2) selon la revendication 1, **caractérisé en ce que** l'armature (5, 5') est inscrite dans au moins une surface cylindrique de plus grande dimension transversale comprise entre 500 µm et 2 mm.

3. Dispositif de prélèvement (5 ou 5', 2) selon la revendication 1 ou 2, **caractérisé en ce que** l'armature (5, 5') présente une face externe (5a, 5'a) et délimite un volume interne, ladite couche polymérique (2a) s'étendant à travers lesdits ajours (5b, 5'b) dudit volume interne à ladite face externe et au-delà de cette dernière.

4. Dispositif de prélèvement (5, 2) selon une des revendications 1 à 3, **caractérisé en ce que** ladite armature (5) présente un unique axe longitudinal de symétrie qui est destiné à être parallèle à celui dudit embout (6), ladite armature conservant une géométrie globalement tubulaire dans lesdites positions.

5. Dispositif de prélèvement (5 ou 5', 2) selon une des revendications précédentes, **caractérisé en ce que** ladite couche polymérique réticulée (2a) présente une viscosité, mesurée par un rhéomètre cône - plan, qui est égale ou supérieure à 100 mPa.s et de préférence comprise entre 150 mPa.s et 5800 mPa.s.

6. Dispositif de prélèvement (5 ou 5', 2) selon une des revendications précédentes, **caractérisé en ce que** ladite armature (5, 5') est de type grillagée, tissée ou tressée, comprenant une multitude desdits ajours (5b, 5'b) séparés deux à deux suivant un pas compris entre 30 µm et 60 µm.

7. Dispositif de prélèvement (5 ou 5', 2) selon la revendication 2 et une des revendications 3 à 6, **caractérisé en ce que** ladite armature (5, 5') présente une épaisseur comprise entre 10 µm et 100 µm, ladite au moins une surface cylindrique présentant une section sensiblement elliptique ou circulaire.

8. Dispositif de prélèvement (5 ou 5', 2) selon la revendication 3 et une des revendications 4 à 7, **caractérisé en ce que** ladite face externe (5a, 5'a) et/ou une face interne de l'armature (5, 5') présente(nt) en outre des creux et/ou des reliefs qui sont distincts desdits ajours (5b, 5'b) et qui présentent des dimensions comprises entre 20 µm et 90 µm.

9. Dispositif de prélèvement (5 ou 5', 2) selon la revendication 3 et une des revendications 4 à 8, **caractérisé en ce que** ladite couche polymérique (2a) forme par rapport à ladite face externe (5a) de l'armature (5, 5') un revêtement externe sensiblement coaxial à cette armature et d'épaisseur comprise entre 50 µm et 300 µm.

10. Dispositif de prélèvement (5 ou 5', 2) selon une des revendications précédentes, **caractérisé en ce que** ladite couche polymérique réticulée (2a) présente un module d'Young, mesuré à partir de tests de compression réalisés avec un rhéomètre, inclusivement compris entre 50 kPa et 270 kPa.

11. Dispositif de prélèvement (5 ou 5', 2) selon une des revendications précédentes, **caractérisé en ce que** ladite armature (5, 5') est noyée dans ladite couche polymérique (2a) sur une partie de sa longueur axiale comprise entre 1 mm et 5 cm, cette couche polymérique présentant un volume compris entre 1 mL et 10 mL.

12. Dispositif de prélèvement (5 ou 5', 2) selon une des revendications précédentes, **caractérisé en ce que** :
- ladite armature (5, 5') est réalisée en un matériau métallique, de préférence de l'acier inoxydable de grade chirurgical, en silicium ou en une matière polymérique telle qu'un silicone,
- ladite couche polymérique (2a) est à base d'au moins un polymère biocompatible et à gélification réversible choisi dans le groupe constitué par les gels d'alginate, les copolymères d'alginate et de poly-L-lysine, le chitosan, l'agarose, la cellulose, le poly(triméthylammonium éthylacrylate méthyl sulfate)-b-poly (acrylamide), le poly(hydroxyéthylmétacrylate (HEMA), le poly(hydroxyéthylmétacrylate-méthyl éthacrylate (HEMA-MMA) et les autres copolymères à base de méthacrylate, les polyéthylèneglycols, les copolymères d'acrylonitrile et de polyéthylèneglycol, les polysaccharides et leurs mélanges, et **en ce que**
- l'armature est pourvue à sa surface de groupements fonctionnels créant des liaisons chimiques entre l'armature et la couche polymérique, de préférence des groupements acide carboxylique ou amine dans le cas où l'armature est métallique pour une liaison avec des groupements hydroxyle de cette couche.

13. Système de prélèvement (6, 7, 5 ou 5', 2) comprenant :
- un embout tubulaire creux (6) de type aiguille ou cathéter qui présente un diamètre interne compris entre 500 µm et 2 mm,
- un dispositif de prélèvement (5 ou 5', 2) inséré dans l'embout et apte à déboucher par coulissement hors d'une extrémité de cet embout en vue d'une mise en contact notamment *in vivo* avec un fluide corporel (12) contenant des cibles biologiques (1) à prélever, et
- un organe de poussée (7) apte à faire coulisser réversiblement ledit dispositif de prélèvement hors dudit embout,
**caractérisé en ce que** ce dispositif est tel que défini à l'une des revendications précédentes, ce dispositif étant optionnellement pourvu d'un moyen de liaison à ladite extrémité de l'embout,
et de préférence **en ce que** ledit organe de poussée (7) est de type seringue et comporte :
- un corps de pompe dans lequel est monté l'embout (6), et
- une tige insérable dans ledit embout pour y faire coulisser ledit dispositif de prélèvement (5 ou 5', 2).

14. Procédé de fabrication d'un dispositif de prélèvement (5 ou 5', 2) selon une des revendications 1 à 12, **caractérisé en ce qu'il** comprend les étapes suivantes :
a) préparation d'un composite polymérique (2) non réticulé incorporant lesdits supports de capture (2b) et ladite couche polymérique (2a) non réticulée les recouvrant,
b) insertion de ladite armature (5, 5') dépourvue de ce composite dans un moule tubulaire, avec optionnellement une liaison de l'armature à une extrémité de prélèvement de l'embout,
c) assemblage de l'embout contenant cette armature dans un organe de prélèvement (7) de type seringue,
d) prélèvement du composite non réticulé préparé en a) par cet organe de prélèvement, pour injecter ce composite à l'intérieur de l'embout au contact de l'armature, puis
e) réticulation dans un bain gélifiant de l'embout qui est rempli du composite non réticulé injecté en d) et que l'on a préalablement extrait de cet organe de prélèvement, pour l'obtention de ladite couche polymérique réticulée solidaire de l'armature.

15. Procédé de fabrication selon la revendication 14, **caractérisé en ce que** l'étape a) comprend :
a1) une dispersion dans une solution tampon aqueuse desdits supports de capture (2b) comprenant des nanoparticules fonctionnalisées magnétiques ou non, puis
a2) un ajout sous agitation à la dispersion obtenue en a1) d'au moins un polymère biocompatible et à gélification réversible, pour l'obtention dudit composite (2) non réticulé dans lequel sont noyées ces nanoparticules.

## Patentansprüche

1. Entnahmevorrichtung (5 oder 5', 2), welche dazu ausgestaltet ist, in ein hohles röhrenförmiges Ansatzstück (6) vom Typ einer Nadel oder eines Katheters eingesetzt zu werden, und um für ein in Kontakt Bringen, insbesondere in vivo, mit einem Körperfluid (12), welches zu entnehmende biologische Ziele (1) enthält, aus dem Ansatzstück hervorzukommen, wobei die Vorrichtung umfasst:
- eine durch Öffnungen (5b, 5'b) mikrostrukturierte Armierung (5, 5'), und
- eine biokompatible und poröse vernetzte Polymerschicht (2a), welche Auffangträger (2b) aufweist, welche dazu ausgestaltet sind, die Ziele aufzufangen, und welche dazu ausgestaltet ist, diese Träger gegenüber dem Fluid zu halten und nur Partikel des diese Ziele beinhaltenden Fluids mit geringerer Größe als eine Größenbegrenzung durchzulassen,
wobei die Polymerschicht alles oder einen Teil der Öffnungen füllt, sodass sie von der Armierung gehalten wird,
**dadurch gekennzeichnet, dass** die Armierung zwischen den Positionen, welche sie in das Ansatzstück eingesetzt ist oder sie aus dem letzteren hervorkommt, im Wesentlichen unverformbar ist.

2. Entnahmevorrichtung (5 oder 5', 2) nach Anspruch 1, **dadurch gekennzeichnet, dass** die Armierung (5, 5') Teil von wenigstens einer Zylinderfläche mit größerer transversaler Abmessung ist, welche zwischen 500 µm und 2 mm enthalten ist.

3. Entnahmevorrichtung (5 oder 5', 2) nach Anspruch 1 oder 2, **dadurch gekennzeichnet, dass** die Armierung (5, 5') eine äußere Seite (5a, 5'a) aufweist und ein inneres Volumen begrenzt, wobei die Polymerschicht (2a) sich durch die Öffnungen (5b, 5'b) von dem inneren Volumen zu der äußeren Seite und über die letztere hinaus erstreckt.

4. Entnahmevorrichtung (5 oder 5', 2) nach einem der Ansprüche 1 bis 3, **dadurch gekennzeichnet, dass** die Armierung (5) eine einzige longitudinale Symmetrieachse aufweist, welche dazu bestimmt ist, parallel zu derjenigen des Ansatzstücks (6) zu sein, wobei die Armierung in denen genannten Positionen eine insgesamt röhrenartige Geometrie beibehält.

5. Entnahmevorrichtung (5 oder 5', 2) nach einem der vorhergehenden Ansprüche, **dadurch gekennzeichnet, dass** die vernetzte Polymerschicht (2a) eine durch ein Kegel-Platte-Rheometer gemessene Viskosität aufweist, welche größer oder gleich 100 mPa.s ist und bevorzugt zwischen 150 mPa.s und 5800 mPa.s enthalten ist.

6. Entnahmevorrichtung (5 oder 5', 2) nach einem der vorhergehenden Ansprüche, **dadurch gekennzeichnet, dass** die Armierung (5, 5') vom Gitter-, Web- oder Flechttyp ist, welcher eine Vielzahl der Öffnungen (5b, 5'b) umfasst, die paarweise um eine Schrittweite beabstandet sind, welche zwischen 30 µm und 60 µm enthalten ist.

7. Entnahmevorrichtung (5 oder 5', 2) nach Anspruch 2 und einem der Ansprüche 3 bis 6, **dadurch gekennzeichnet, dass** die Armierung (5, 5') eine Dicke aufweist, welche zwischen 10 µm und 100 µm enthalten ist, wobei die wenigstens eine Zylinderfläche einen im Wesentlichen elliptischen oder kreisförmigen Querschnitt aufweist.

8. Entnahmevorrichtung (5 oder 5', 2) nach Anspruch 3 und einem der Ansprüche 4 bis 7, **dadurch gekennzeichnet, dass** die äußere Seite (5a, 5'a) und/oder eine innere Seite der Armierung (5, 5') außerdem Vertiefungen und/oder Erhebungen aufweist/aufweisen, welche separat von den Öffnungen (5b, 5'b) sind und welche Abmessungen aufweisen, welche zwischen 20 µm und 90 µm enthalten sind.

9. Entnahmevorrichtung (5 oder 5', 2) nach Anspruch 3 und einem der Ansprüche 4 bis 8, **dadurch gekennzeichnet, dass** die Polymerschicht (2a) mit Bezug auf die äußere Seite (5a) der Armierung (5, 5') eine äußere Beschichtung bildet, welche im Wesentlichen koaxial zu dieser Armierung ist und eine Dicke aufweist, welche zwischen 50 µm und 30 µm enthalten ist.

10. Entnahmevorrichtung (5 oder 5', 2) nach einem der vorhergehenden Ansprüche, **dadurch gekennzeichnet, dass** die vernetzte Polymerschicht (2a) einen ausgehend von mit einem Rheometer realisierten Kompressionstests gemessenen Young-Modul aufweist, welcher zwischen einschließlich 50 kPa und 270 kPa enthalten ist.

11. Entnahmevorrichtung (5 oder 5', 2) nach einem der vorhergehenden Ansprüche, **dadurch gekennzeichnet, dass** die Armierung (5, 5') in der Polymerschicht (2a) auf einem Teil ihrer axialen Länge, welcher zwischen 1 mm und 5 cm enthalten ist, eingebettet ist, wobei diese Polymerschicht ein Volumen aufweist, welches zwischen 1 ml und 10 ml enthalten ist.

12. Entnahmevorrichtung (5 oder 5', 2) nach einem der vorhergehenden Ansprüche, **dadurch gekennzeichnet, dass**:
- die Armierung (5, 5') realisiert ist aus einem metallischen Material, bevorzugt rostfreier Stahl von chirurgischer Qualität, aus Silizium oder aus einem polymeren Material, wie zum Beispiel Silikon,
- die Polymerschicht (2a) auf wenigstens einem Polymer basiert, welches biokompatibel und mit reversibler Gelierung ist, ausgewählt aus der Gruppe, welche gebildet ist durch Alginatgelen, Copolymeren von Alginsäure und Poly-L-Lysin, Chitosan, Agarose, Cellulose, Poly(Trimethylammonium-Ethylacrylat-Methyl-Sulfat)-b-Poly(Acrylamid), Poly(Hydroxyethylmetacrylat (HEMA)), Poly-(Hydroxymethylmetacrylat-Methylethacrylat (HEMA-MMA)) und andere Copolymere auf Basis von Metacrylat, Polyethylenglykole, Copolymere von Akrylonitril und Polyethylenglykolen, Copolymeren von Akrylonitril und Polyethylenglykol, Polysaccharide, und ihre Mischungen, und dass
- die Armierung an ihrer Oberfläche mit funktionellen Gruppen versehen ist, welche chemische Verbindungen zwischen der Armierung und der Polymerschicht erzeugen, bevorzugt mit Carboxylsäure- oder Aminosäuregruppen, wenn die Armierung metallisch ist für eine Verbindung mit den Hydroxylgruppen dieser Schicht.

13. Entnahmesystem (6, 7, 5 oder 5', 2) umfassend:
- ein hohles röhrenförmiges Ansatzstück (6) vom Typ einer Nadel oder eines Katheters, welches einen inneren Durchmesser aufweist, der zwischen 500 µm und 2 mm enthalten ist,
- eine Entnahmevorrichtung (5 oder 5', 2), welche in das Ansatzstück eingesetzt ist und dazu ausgestaltet ist, für ein in Kontakt Bringen, insbesondere in vivo, mit einem Körperfluid (12), welches zu entnehmende biologische Ziele (1) enthält, durch Gleiten aus einem Ende dieses Ansatzstücks hervorzukommen und
- ein Schiebeelement (7), welches dazu ausgestaltet ist, die Entnahmevorrichtung reversibel aus dem Ansatzstück heraus gleiten zu lassen,
**dadurch gekennzeichnet, dass** die Vorrichtung so wie in einem der vorhergehenden Ansprüche definiert ist, wobei diese Vorrichtung optional mit einem Mittel zur Verbindung mit dem Ende des Ansatzstücks versehen ist,
und bevorzugt, dass das Schiebeelement (7) vom Spritzentyp ist und umfasst:
- einen Pumpkörper, in welchem das Ansatzstück (6) angebracht ist, und
- einen Stempel, welcher in dem Ansatzstück einsetzbar ist, um die Entnahmevorrichtung (5 oder 5', 2) darin gleiten zu lassen.

14. Verfahren zur Herstellung einer Entnahmevorrichtung (5 oder 5', 2) nach einem der Ansprüche 1 bis 12, **dadurch gekennzeichnet, dass** es die folgenden Schritte umfasst:
a) Herstellen eines nicht-vernetzten polymeren Verbunds (2), welcher die Auffangträger (2b) und die sie bedeckende nicht-vernetzte Polymerschicht (2a) beinhaltet,
b) Einsetzen der Armierung (5, 5') ohne diesen Verbund in eine röhrenförmige Form, mit optional einer Verbindung der Armierung mit einem Entnahmeende des Ansatzstücks,
c) Anbringen des diese Armierung beinhaltenden Ansatzstücks in einem Entnahmeelement (7) vom Spritzentyp,
d) Entnahme des bei a) hergestellten nicht-vernetzten Verbunds durch dieses Entnahmeelement, um diesen Verbund in Kontakt mit der Armierung in das Innere des Ansatzstücks einzuführen, und dann
e) Vernetzen in einen Gelierbad des Ansatzstücks, welches mit dem bei d) eingeführten und zuvor aus diesem Entnahmeelement extrahierten nicht-vernetzten Verbund gefüllt ist, um die mit der Armierung verbundene vernetzte Polymerschicht zu erhalten.

15. Herstellungsverfahren nach Anspruch 14, **dadurch gekennzeichnet, dass** der Schritt a) umfasst:
a1) Dispergieren der Auffangträger (2b), welche funktionalisierte magnetische oder nicht-magnetische Nanopartikel umfassen in einer wässerigen Pufferlösung, und dann
a2) ein Zuführen unter Rühren zu der bei a1) erhaltenen Dispersion von wenigstens einem Polymer, welches biokompatibel und mit reversibler Gelierung ist, um den nicht-vernetzten Verbund (2) zu erhalten, in welchem diese Nanopartikel eingebettet sind.

## Claims

1. Sampling device (5 or 5', 2) adapted to be inserted into a hollow tubular endpiece (6) of the needle or catheter type, and to emerge from the endpiece with a view to contact, particularly *in vivo,* with a bodily fluid (12) containing biological targets (1) to be sampled, the device comprising:
- a framework (5, 5') microstructured by openings (5b, 5'b), and
- a biocompatible and porous crosslinked polymer layer (2a) which comprises capture supports (2b) adapted to capture the said targets and which is adapted to retain these supports from the fluid and to let through only fluid particles including these targets with a size of less than a cutoff size,
the said polymer layer filling all or some of the said openings, so as to be retained by the said framework,
**characterized in that** the said framework is substantially undeformable between positions in which it is inserted into the endpiece and in which it emerges from the latter.

2. Sampling device (5 or 5', 2) according to Claim 1, **characterized in that** the framework (5, 5') is circumscribed by at least one cylindrical surface having a largest transverse dimension of between 500 µm and 2 mm.

3. Sampling device (5 or 5', 2) according to Claim 1 or 2, **characterized in that** the framework (5, 5') has an external face (5a, 5'a) and delimits an internal volume, the said polymer layer (2a) extending through the said openings (5b, 5'b) from the said internal volume to the said external face and beyond the latter.

4. Sampling device (5 or 5', 2) according to one of Claims 1 to 3, **characterized in that** the said framework (5) has a single longitudinal symmetry axis which is intended to be parallel to that of the said endpiece (6), the said framework maintaining an overall tubular geometry in the said positions.

5. Sampling device (5 or 5', 2) according to one of the preceding claims, **characterized in that** the said crosslinked polymer layer (2a) has a viscosity, measured by a cone and plate rheometer, which is equal to or greater than 100 mPa.s and preferably lies between 150 mPa.s and 5800 mPa.s.

6. Sampling device (5 or 5', 2) according to one of the preceding claims, **characterized in that** the said framework (5, 5') is of the latticed, woven or plaited type, comprising a multitude of the said openings (5b, 5'b), separated in pairs by a pitch of between 30 µm and 60 µm,

7. Sampling device (5 or 5', 2) according to Claim 2 and one of Claims 3 to 6, **characterized in that** the said framework (5, 5') has a thickness of between 10 µm and 100 µm, the said at least one cylindrical surface having a substantially elliptical or circular cross section.

8. Sampling device (5 or 5', 2) according to Claim 3 and one of Claims 4 to 7, **characterized in that** the said external face (5a, 5'a) and/or an internal face of the framework (5, 5') furthermore has or have indentations and/or reliefs which are separate from the said openings (5b, 5'b) and which have dimensions of between 20 µm and 90 µm.

9. Sampling device (5 or 5', 2) according to Claim 3 and one of Claims 4 to 8, **characterized in that** the said polymer layer (2a) forms, with respect to the said external face (5a) of the framework (5, 5'), an external coating substantially coaxial with this framework and having a thickness of between 50 µm and 300 µm.

10. Sampling device (5 or 5', 2) according to one of the preceding claims, **characterized in that** the said crosslinked polymer layer (2a) has a Young's modulus, measured on the basis of compression tests carried out with a rheometer, of between 50 kPa and 270 kPa inclusive.

11. Sampling device (5 or 5', 2) according to one of the preceding claims, **characterized in that** the said framework (5, 5') is embedded in the said polymer layer (2a) over a part of its axial length lying between 1 mm and 5 cm, this polymer layer having a volume of between 1 ml and 10 ml.

12. Sampling device (5 or 5', 2) according to one of the preceding claims, **characterized in that**:
- the said framework (5, 5') is made of metallic material, preferably stainless steel of surgical grade, silicon or a polymer material such as a silicone,
- the said polymer layer (2a) is based on at least one biocompatible polymer with reversible gelling, selected from the group consisting of alginate gels, copolymers of alginate and poly-L-iysine, chitosan, agarose, cellulose, poly(trimethylammonium ethylacrylate methyl sulfate)-b-poly(acrylamide), poly(hydroxyethylmethacrylate (HEMA), poly(hydroxyethylmethacrylate-methyl methacrylate (HEMA-MMA) and other copolymers based on methacrylate, polyethylene glycols, copolymers of acrylonitrile and polyethylene glycol, polysaccharides and mixtures thereof, and **in that**
- the framework is provided on its surface with functional groups creating chemical bonds between the framework and the polymer layer, preferably carboxylic acid or amine groups in the case in which the framework is metallic for bonding with hydroxyl groups of this layer.

13. Sampling system (6, 7, 5 or 5', 2) comprising:
- a hollow tubular endpiece (6) of the needle or catheter type, which has an internal diameter of between 500 µm and 2 mm,
- a sampling device (5 or 5', 2) inserted into the endpiece and capable of emerging by sliding from an end of this endpiece with a view to contact, particularly *in vivo,* with a bodily fluid (12) containing biological targets (1) to be sampled, and
- a thrust member (7) capable of making the said sampling device slide reversibly out of the said endpiece,
**characterized in that** this device is as defined in one of the preceding claims, this device optionally being provided with a means for connection to the said end of the endpiece, and preferably **in that** the said thrust member (7) is of the syringe type and comprises:
- a pump body, in which the endpiece (6) is mounted, and
- a rod which can be inserted into the said endpiece in order to make the said sampling device (5 or 5', 2) slide therein.

14. Method for manufacturing a sampling device (5 or 5', 2) according to one of Claims 1 to 12, **characterized in that** it comprises the following steps:
a) preparation of an uncrosslinked polymer composite (2) incorporating the said capture supports (2b) and the said uncrosslinked polymer layer (2a) covering them,
b) insertion of the said framework (5, 5'), without this composite, into a tubular mold, optionally with connection of the framework to a sampling end of the endpiece,
c) assembly of the endpiece containing this framework in a sampling member (7) of the syringe type,
d) take-up of the uncrosslinked composite prepared in a) by this sampling member, in order to inject this composite inside the endpiece in contact with the framework, then
e) crosslinking in a gelling bath of the endpiece which is filled with the uncrosslinked composite injected in d) and which has previously been extracted from this sampling member, in order to obtain the said crosslinked polymer layer fixed to the framework.

15. Manufacturing method according to Claim 15, **characterized in that** step a) comprises:
a1) dispersion in an aqueous buffer solution of the said capture supports (2b) comprising magnetic or non-magnetic functionalized nanoparticles, then
a2) addition under agitation to the dispersion obtained in a1) of at least one biocompatible polymer with reversible gelling, in order to obtain the said uncrosslinked composite (2) in which these nanoparticles are embedded.
